# EUROPEAN PATENT APPLICATION

(11) **EP 2 172 489 A1**
(43) Date of publication of application: **07.04.2010**
(21) Application number: 08164360.3
(22) Date of filing: 15.09.2008
(51) Int. Cl.: C08B 30/00, C12N 9/10

(54) **Method for altering starch using a microbial branching enzyme**

(71) Applicant: Nederlandse Organisatie voor Toegepast- Natuurwetenschappelijk Onderzoek TNO, 2628 VK Delft (NL)
(72) Inventor: Van der Maarel, Marc Jos Elise Cornelis, 9753 CC Haren (NL); Palomo i Reiach, Marta, 9725 GZ Groningen (NL); Booiman, Thijs, 7011 VD Gaanderen (NL); Dijkhuizen, Lubbert, 9472 RB Zuidlaren (NL)
(74) Representative: Hatzmann, Martin

(57) **Abstract**

The invention relates to the field of the production and application of starch. Provided is a method for producing altered starch, comprising treating amylose-containing starch with a branching enzyme (EC 2.4.1.18) of microbial origin, said branching enzyme having a substantially higher activity on amylose than on amylopectin. Also provided is the use of the branching enzyme having a preference for amylose over amylopectin, to alter least one property of amylose-containing starch, including reducing the retrogradation, a reducing the viscosity and/or reducing the glycemic index of the starch

## Description

The invention relates to the field of the production and application of starch. The invention particularly relates to methods and means for producing starch having at least one altered characteristic, comprising treating the starch with a branching enzyme of microbial origin.

Starch forms a major component of man and animal diets, supplying them with a large portion of their carbohydrate intake. Starch is also used industrially in the production of inter alia, paper, textiles, plastics and adhesives, as well as providing the raw material for the production of fermentable sugars used in industrial fermentations. Starch from different botanical origin has preferred uses. On a world scale, starch producing crops such as wheat, maize, rice and potatoes are agriculturally and economically by far the most important. The type of starch will affect the quality of a processed product and the profitability of the processed crop. In addition, the quantity and quality of starch present in the harvested organ of a plant will affect the gross yield and the processing efficiency.

Starch consists of two glucose polymers, i.e. amylose with alpha, 1-4 glycosidic linkages and amylopectin, containing about 95-97% alpha, 1-4 and 3 to 5% alpha, 1-6 glycosidic linkages. Amylopectin contains short and long chains, the short chains ranging from 5-30 glucose units and the long chains ranging from 30-100 glucose units, or more. Starch with various amounts of amylose and amylopectin are found in different plants. Typically, starch contains 10-25% amylose, the remainder being amylopectin. It is thought that the ratio of amylose to amylopectin and the distribution of short to long chains in the amylopectin fraction affect the physical properties of starch, e.g. thermal stabilisation, retrogradation and viscosity. These properties also affect the utility of starch, as mentioned above.

Starch occurs in the form of granules that swell and burst open upon heating, resulting in the release of the amylose and amylopectin into the solution. Upon cooling of this solution, the linear amylose molecules will interact with each other to form a firm network (gelling) that cannot be dissolved. This process is called retrogradation and causes a number of problems in downstream starch processing. One problem resides in the fact that only a limited dry matter content can be used in the manufacture of fermentable sugars from starch, a process performed on a large scale in e.g. the production of bioalcohol or syrups (maltodextrin, maltose, glucose) for the food industry. Typically, an aqueous suspension of starch is heated to elevated temperatures by a process called jet cooking, followed immediately by the addition of a thermostable amylase enzyme to partially degrade amylose, thereby liquefying the starch gel. The liquefaction process usually is performed at about 90°C to avoid a high viscosity. The liquefied starch is further degraded (the saccharification) to maltodextrins using one or more other enzymes at about 65°C. The maximal dry matter content of the starch suspension used as starting material is about 35%; higher values result in a too high viscosity and/or undesired retrogradation . A major drawback of the above mentioned process of jet cooking and liquefaction using an amylase treatment resides in high energy costs; the liquefaction step consumes about 8 to 10% of the total energy required to convert starch into a fermentable sugar.

Methods to minimize starch retrogradation are known in the art and typically involve the genetic modification of a starch-producing plant such that no or very little amylose is formed. Naturally occurring or genetically modified plants devoid of amylose production are referred to as "waxy variants". Waxy variants of potato starch and maize starch are known. For example, waxy corn starch lacks amylose-type starch and this starch has unique properties (low viscosity and high stability). Also, most mutations in the waxy locus of maize, which encodes starch granule bound synthase I (GBSSI), result in plants which produce much reduced amylose. When no functioning GBSSI is synthesised in the homozygous waxy mutant it also lacks amylose. Although starch derived from waxy variants has advantageous properties, its practical use is hampered by factors as limited availability (only potato and maize), public aversion against the use of GMO, plant breeder's rights, and, in particular, its prize; the cost of one kilogram of waxy starch is about 2-5-fold higher than that of regular starch.

An object of the present invention is to provide methods and means for producing starch having at least one altered characteristic, in particular starch having improved resistance to retrogradation. Preferably, the method is both economically attractive and versatile, e.g. applicable to any source of plant starch.

It was found that at least some of these goals can be met by using microbial enzymes having a unique substrate preference, allowing for the selective removal of amylose from starch. More specifically, it was found that a glycogen branching enzyme (EC 2.4.1.18) of microbial origin, said branching enzyme having a substantially higher activity on amylose than on amylopectin, can be used to convert essentially any type of amylose-containing starch into a "waxy-type" starch in a cost-and time effective manner.

Accordingly, the invention relates to a method for providing altered starch, comprising treating starch with a branching enzyme (EC 2.4.1.18) of microbial origin, said branching enzyme having a substantially higher activity on amylose than on amylopectin. As used herein, the expression "substantially higher activity on amylase than on amylopectin" is meant to indicate that the enzyme displays a relative substrate preference for amylose when assayed for activity towards amylose or amylopection in a suitable enzyme activity assay, e.g. an iodine-staining assay. For example, the substrate preference of said branching enzyme for amylose is at least 2-fold, preferably at least 4-fold, more preferably at least 5-fold higher than for amylopectin.

In one embodiment, the branching enzyme is a glycogen branching enzyme. Glycogen branching enzymes (GBE) or 1,4-α-glucan branching enzymes (EC 2.4.1.18) catalyze the formation of branching points in glycogen by hydrolyzing α-1,4 linkages and the formation of α-1,6 linkages. Glycogen branching enzymes are known to act on both amylose and amylopectin. Most branching enzymes are reported to be part of the glycoside hydrolase family 13 (GH-13). Glycogen branching enzymes from Eukaryotes are reported in subfamily 8 while those from prokaryotes are part of subfamily 9.

Typically, a method of the invention for providing altered starch comprises bringing at least one branching enzyme into contact with gelatinized and/or liquified starch or with an aqueous starch suspension. Preferably, the enzyme treatment is performed at a temperature up to the gelatinization temperature of the starch, i.e. between 60 and 75-80°C. This is at a lower temperature, and hence needs less energy, than in the currently used methods for liquifying starch using jet-cooking followed by amylase treatment. Also, the dry matter content of the suspension to be treated according to a method of the prior art should not be above around 35% to avoid a high viscosity and rapid retrogradation. The lower the dry matter content of the starting material, the more water needs to be removed afterwards to obtain a dried product. In contrast, a method of the invention employing a branching enzyme with the advantageous substrate preference for amylose allows for an efficient removal (i.e. branching) of the linear amylose, thereby reducing the viscosity of the starch-containing mixture. As a consequence, the dry matter content of the starting suspension in a method of the invention can be above 35%. This reduces the amount of water that needs to be removed, resulting in a reduction of the time and energy needed to obtain a dried product. In one embodiment, the dry matter content of the suspension is from about 10 to 50%, preferably 35-50%.

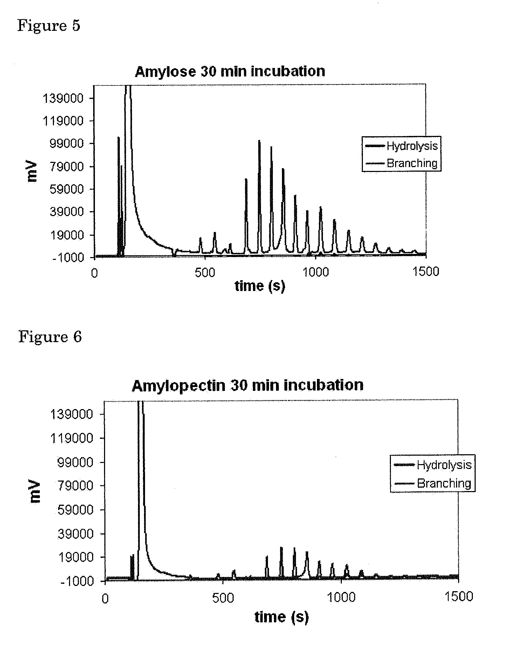

The total incubation time with branching enzyme can vary, depending for instance on the substrate and/or enzyme used or on process parameters such as incubation temperature, dry matter content, and the like. In general, the incubation time should be sufficient to remove amylose while minimizing the branching activity towards amylopectin. The skilled person will be able to determine the optimal incubation period by routine analysis of the product(s) formed at various time points using methods known in the art, for example by determining side-chain distribution and degree of branching. It was found that an incubation time ranging from a few minutes to a few hours with an "amylose-specific" enzyme according to the invention is sufficient to obtain the desired selective removal of amylose from amylose-containing starch. As shown in Figures 5 and 6, a 30 minute incubation time resulted in amylose branching up to a degree of branching of 4% while the degree of branching of amylopection after said incubation was essentially unchanged as compared to that of the starting material. In a preferred embodiment, a method of the invention comprises treating amylose-containing starch with a branching enzyme (EC 2.4.1.18) of microbial origin, said branching enzyme having a substantially higher activity on amylose than on amylopectin, during a time period of 5 min to 5 hours, more preferably 5 min to 3 hours, such as 5 min to 1 hour. In a specific aspect, the incubation time ranges from about 10 min to about 45 min.

As will be clear to a person skilled in the art, the amount of branching enzyme required to obtain the desired result will depend on the amount of substrate to be treated. Thus, higher concentrations of starch will generally need more enzyme. The amount needed can be readily established using pilot experiments.

As said above, a method as provided herein typically involves contacting or treating an aqueous suspension of amylose-containing starch with the branching enzyme at a temperature up to the gelatinization temperature of starch. It is thus preferred that the enzyme(s) used are active and stable at said temperature. In one embodiment, the enzyme displays optimal activity in the range of between about 50 and 80 °C, more preferably between about 60 and 70°C. Furthermore, thermal stability up to at least 70°C, more preferably at least 80°C is preferred. Enzymes of particular interest for practicing the invention include branching enzymes that originate from thermophilic microorganisms. Of course, natural or artificial mutant enzymes ("thermostable variants") are also encompassed.

In a preferred embodiment, the present invention utilizes a branching enzyme that belongs to the family 57 of glycoside hydrolases (GH-57). The glycoside hydrolase family 57(GH-57 family) was established in 1996 on the basis of two amylase sequences that were lacking the conserved sequence region characteristics of GH-13 family α-amylases. Most of the GH-57 family enzymes are thermostable and originate from extremophiles. Individual members of the GH-57 family display a high sequence and length diversity, sequence length can vary from 400 up to 1500 residues. Therefore sequences cannot be aligned with routine alignment programs. With manual alignments five conserved sequence regions were found characteristic for the GH-57 family. The two proposed catalytic residues are also found in these conserved sequence regions. For the glycogen branching enzymes an extra conserved region was found. Enzymes found in the GH-57 family are multidomain proteins. They all share a catalytic (β/α)7-barrel with a putative nucleophile (Glutamate) and a putative proton donor (Aspartate) at 6-strand 4 and 6-strand 7.

In a specific aspect, a method provided herein makes use of glycogen branching enzyme derived from *Thermus thermophilus.* One of the orthologs of the novel glycogen branching enzyme found by Murakami *et al.* was found in the *Thermus thermophilus* strain HB8 (Journal of Bacteriology (2006) 188:5915-59242), which is a Gram negative bacterium with an optimum growth temperature of 65°C. *Thermus thermophilus* glycogen was found to be highly branched and having an average chain length of 7 glucose residues (Boeck B., Schinzel R. (1998) Microbiol. 149, 171-176).

It will be appreciated that a method according to the invention is applicable to reduce the amylose content of any type of starch that contains, or is suspected to contain, amylose. The starch can be a native plant starch, such as potato starch, maize starch, wheat starch, tapioca starch or rice starch. The starch may be derived from a GMO or a non-GMO plant variant. Although the invention provides an economically alternative for the conventional yet costly "waxy starch" approach, application to remove trace amounts of amylose from waxy variants is also envisaged.

A further embodiment relates to the use of a branching enzyme (EC 2.4.1.18) of microbial origin, said branching enzyme having a substrate preference for amylose over amylopectin, to alter at least one property of amylose-containing starch. Exemplary enzymes and preferred enzymes are described herein above. Briefly, the branching enzyme preferably originates from a thermophilic micro-organism or the enzyme is a thermostable variant. The branching enzyme may belong to the family 57 of glycoside hydrolases (GH-57), in particular the enzyme is derived from *Thermus thermophilus.*

In a specific aspect, the branching enzyme is used to improve at least one starch property that is beneficial in terms of downstream starch processing, such as reducing the retrogradation and/or reducing the viscosity. A further aspect involves the alteration of a property related to the nutritional characteristics of starch, such as reducing the glycemic index (GI) of the starch by the selective removal of amylose.

### LEGENDS TO THE FIGURES

Figure 1: SDS-PAGE analysis showing the overexpression in E. coli and subsequent purification of the *Thermus thermophilus* glycogen branching enzyme (GBE). In lane 1: molecular weight marker, lane 2: 5 hours post IPTG induction sample, lane 3: pellet after sonification, lane 4: supernatant after sonication, lane 5: supernatant after 70°C incubation, lane 6: 9µg of *T. thermophilus* GBE after HIS-tag purification and lane 7: of *T. thermophilus* GBE after Anion exchange chromatography.
Figure 2: Temperature-dependency of the *T. thermophilus* GBE.
Figure 3: Thermostability of the *T. thermophilus* GBE.
Figure 4: Branching enzyme activity towards amylose and amylopectin as determined by iodine-staining.
Figure 5: Amylose side-chain distribution after a 30 minutes incubation of amylose with the *T. thermophilus* GBE. The polymerization degree (DP) of the peaks is marked with a number.
Figure 6: Amylopectin side-chain distribution after a 30 minute incubation of amylopectin with the *T. thermophilus* GBE. The DP of the peaks is marked with a number.

### EXPERIMENTAL SECTION

### Materials and Methods

**Glycogen branching enzyme cloning, overexpression and purification.**

### Enzyme cloning

The gene encoding the glycogen branching enzyme of *Thermus thermophilus* HB8 (GenBank accession number AP008226.1) was amplified by PCR using the following primers:
5'-GATGCATCATATGGCGCGCTTCGCCCTGGTCCTCCACGCCCACCTCCCC-3' and
5'-GCTACTAAGATCTTCACGCCTCCCGGAAAAGGTAGAGGCGGGGGTCGGCC-3'.

The PCR product was cloned in the pET15b expression vector and subsequently transformed to *E. coli,* using standard molecular biology techniques. The pET-15b vector possesses a Histidine Tag at the N-terminus, a subsequent thrombin cleavage site and three cloning sites.

### Enzyme overexpression

A 1 liter culture of *E. coli* BL21DE3(STAR) cells transformed with the vector containing the nucleic acid sequence encoding the *T. thermophilus* glycogen branching enzyme was grown in LB broth containing 100µg/mL ampicilin. At an OD600 of 0.6 the bacteria were induced with ImM of IPTG. The cells where grown for an extra 5 hours prior to harvesting. The cells were harvested by centrifuging the culture liquid for 10 minutes at 5,000rpm at 4°C. The supernatant was discarded and the cell pellet washed with 50 mM phosphate buffer pH 8. After the washing the cells were centrifuged for 10 minutes at 5,000 rpm at 4°C. The supernatant was discarded and the cell pellet resuspended in 50 mM Phosphate buffer pH 8. The cell suspension was sonicated by 8 cycles of 15 seconds on and 30 seconds off to disrupt the cells. The cell lysate was centrifuged for 30 minutes at 15,000 rpm at 4°C. The cell free extract was then used for purification.

### Enzyme purification

The cell free extract was incubated at 70°C for 30 min and centrifuged for 30 minutes at 15,000 rpm at 4°C to remove heat-labile proteins from the host cells. The proteins in the cell free extract were purified further with HIS-tag purification and ion exchange chromatography. His-tag purification was done on a HiTrap™ 1 mL chelating HP column (GE Healthcare) with 0.1M Ni(II)sulfate hexahydrate. A gradient with a 50mM phosphate pH 8 buffer with an end concentration of 400mM Imidazol was used to elute the protein of the column. For the anion exchange a HiTrap™ 1 mL Q HP column (GE Healthcare) was used. The protein was eluted from the column with a gradient of a 20mM Tris.HCl pH 8 buffer with an end concentration of 1M NaCl. After purification the proteins were dialyzed into a 25 mM phosphate buffer pH8. The protein concentration was determined with a Nanodrop® ND-1000 UV-Vis spectrophotometer at 280nm.

### SDS-PAGE electrophoresis

The protein samples were analyzed on a SDS-PAGE gel with a 10% acrylamide running gel. The gels were stained with Bio-safe™ Coomassie blue.

### Iodine-staining assay

The iodine-staining assay monitors changes in amylose structure as a result of branching and/or hydrolytic activity. The amylose or amylopectin forms a complex with the iodine and the absorption of this complex is measured. For the assay 50µl of appropriately diluted branching enzyme was incubated at 65°C with either 150 µl 0.125% amyloseV (AVEBE, Veendam, The Netherlands) or 0.25% (w/v) amylopectin (Sigma-Aldrich) in 25mM phosphate buffer pH6.5. Every minute a samples of 15 µl was taken in a 5 minutes reaction and transferred to a 96 wells microtiterplate. The reaction was stopped by adding 1.5 µl 1M HCl to the samples. 150µl freshly made iodine reagent was added to the samples and the OD was measured at 660nm (amylase) or 530 nm (amylopectin) to determiner the specific activity of the enzyme. One unit of activity is defined as the amount of enzyme that can decrease the A₆₆₀ or A₅₃₀ of respectively a amylose or amylopectin-iodine complex by 1% per minute at optimum pH and temperature. To determine the thermostability, the glycogen branching enzyme was incubated for 1 hour at different temperatures and afterwards centrifuged for 1 min at 12,000 rpm. Residual activity of the supernatant was then measured at 65°C.

### Product characterization

### Side-chain distribution

For the product analysis of the glycogen branching enzyme, 20 ml 0.25% (w/v) amylose and 0.25% (w/v) amylopectin was incubated at 65°C pH6.5 with 0.125 mg of enzyme for 144 hours. At 73 hours of incubation, an additional 0.06 mg of enzyme was added. 3 ml samples were taken at different time point and 1.5 ml of the sample was debranched with 225 µl 1M acetate buffer pH3.8 and 20 µl isoamylase. These samples were incubated for 20 hours at 40°C. All samples were freeze-dried and dissolved in 90% DMSO with a final concentration of 0.25 mg/mL. Samples were analyzed on a high performance anion exchange chromatography system with a pulsed amperometric detection system (HPAECPAD)

### Results

### Glycogen branching enzyme overexpression and purification

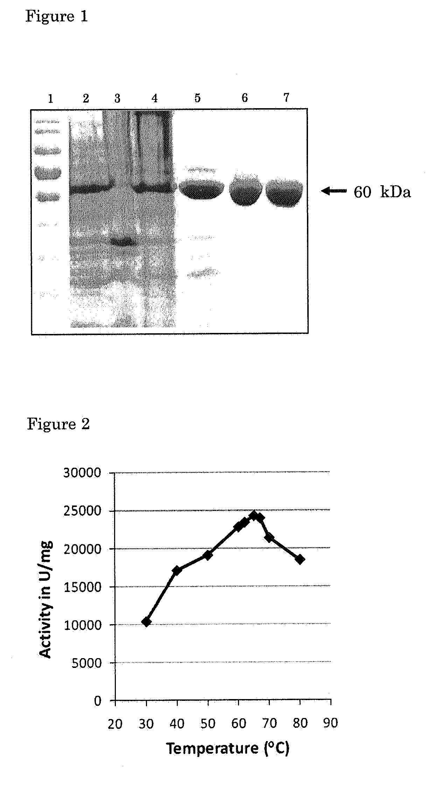

The enzyme was overexpressed in a 1 liter culture with a yield of 7 mg. Figure 1 (lane 2) shows that five hours after induction with IPTG a protein band of 60 KDa is observed; the size of this band corresponds with the predicted molecular weight of the *T. thermophilus* glycogen branching enzyme. In lane 4 it is visible that after sonification and centrifugation the glycogen branching enzyme is located in the supernatant. This supernatant was then further purified by applying a 70°C incubation. At which most of the thermolabile proteins were lost (Figure 1, lane 5). The protein was purified further by performing a HIS-tag purification(Fig. 1 lane 6) The protein was further purified by anion exchange chromatography, resulting in a pure protein preparation.

### Characterization of the glycogen branching enzyme

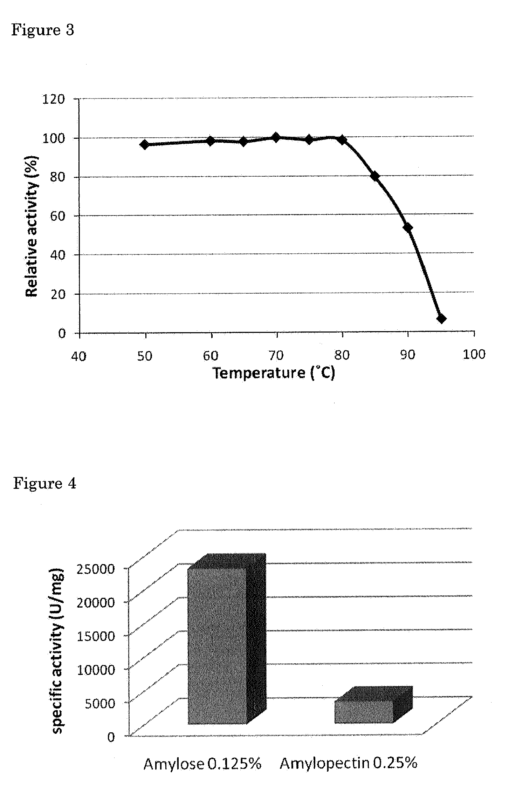

A temperature optimum of 65°C was found (Fig. 2). The enzyme is thermostable for at least one hour up to 80°C (Figure 3).

### Substrate preference

Figure 4 shows that the enzyme has a substantially lower activity on amylopectin compared to amylose. A specific activity of 25,000 units per mg of enzyme was found on amylase, while the specific activity on amylopectin was about 3,700 units/mg of protein.

### Glycogen branching enzyme product characterization

The side-chain distributions of different glycogen branching enzyme products were determined with either amylose or amylopectin as a substrate.

The product obtained from amylose after 144 hours of incubation had side chains with a degree of polymerization (DP) of 4 until 16 with a majority of DP 6 chains. For the hydrolytic activity the enzyme produces chains with a length of DP 3 until 13 with a majority of DP 7 and DP 11 chains. Interestingly, after a short incubation period of 30 minutes no hydrolytic activity towards amylose is observed (Figure 5). Thus, a short incubation time allows for an increase in side chains while no free oligosaccharides are formed.

For the product obtained from amylopectin after 144h of incubation, it was observed that the enzyme transfers side chains of DP 4 until 12. The majority of chains transferred are DP 6 and DP 7. Also, hydrolytic activity on amylopectin is observed. For the hydrolytic activity, the enzyme produces chains of DP 4 until DP 11 with a majority of DP 7 and DP 11 . Also incubation of 30 minute on amylopectin did not give hydrolysis of the amylopectin( Figure 6). With respect to the branching activity, side-chains of DP 5 until DP 13 are transferred with a majority of DP 6 and DP 7 chains. After 30 minutes of incubation the enzyme seems to be slightly more transferring longer chains from DP 11 until DP 12 compared to the amylopectin end product, but it should be noted that method used to determine the side-chain distributions is not quantitative.

## Claims

1. A method for providing altered starch, comprising contacting or treating amylose-containing starch with a branching enzyme (EC 2.4.1.18) of microbial origin, said branching enzyme having a substantially higher activity on amylose than on amylopectin.

2. Method according to claim 1, wherein the substrate preference of said branching enzyme for amylose is at least 2-fold, preferably at least 4-fold, more preferably at least 5-fold higher than for amylopectin

3. Method according to claim 1 or 2, wherein the branching enzyme belongs to the family 57 of glycoside hydrolases (GH-57).

4. Method according to any one of claims 1 to 3, wherein the branching enzyme originates from a thermophilic microorganism or wherein the enzyme is a thermostable variant.

5. Method according to any one of claims 1-4, wherein the enzyme is derived from *Thermus thermophilus.*

6. Method according to any one of the above claims, comprising treating said branching enzyme with gelatinized and/or liquified starch or an aqueous starch suspension.

7. Method according to claim 6, wherein the dry matter content of the suspension is from about 10 to 50%, preferably 35-50%.

8. Method according to any one of the above claims, wherein said treatment is performed at a temperature up to the gelatinization temperature of the starch, preferably between around 60 to around 70°C.

9. Method according to any one of the above claims, wherein said treatment is performed during a time period ranging from a few minutes to a few hours, preferably 5 min to 5 hours, more preferably 5 min to 3 hours, more preferably 5 min to 1 hour, preferably 10 min to 45 min.

10. Method according to any one of the above claims, wherein said starch is a native starch, such as potato starch, maize starch, wheat starch, tapioca starch, rice starch, waxy variants, GMO variants, non-GMO starch.

11. Use of a branching enzyme (EC 2.4.1.18) of microbial origin, said branching enzyme having a substrate preference for amylose over amylopectin, to alter least one property of amylose-containing starch.

12. Use according to claim 11, wherein altering said property comprises improving a property that is beneficial in terms of downstream starch processing, such as reducing the retrogradation and/or reducing the viscosity.

13. Use according to claim 11, wherein altering said property comprises improving a nutritional property, such as reducing the glycemic index of the starch.

14. Use according to any one of claims 11-13, wherein the branching enzyme belongs to the family 57 of glycoside hydrolases (GH-57).

15. Use according to any one of claims 11-14, wherein the branching enzyme originates from a thermophilic microorganism or wherein the enzyme is a thermostable variant.

16. Use according to any one of claims 11-15, wherein the enzyme is derived from *Thermus thermophilus.*
